# EUROPEAN PATENT APPLICATION

(11) **EP 2 093 568 A1**
(43) Date of publication of application: **26.08.2009**
(21) Application number: 08380054.0
(22) Date of filing: 21.02.2008
(51) Int. Cl.: G01N 33/574, C12Q 1/68

(54) **Brca1 mRNA expression predicts survival in patients with bladder cancer treated with neoadjuvant cisplatin-based chemotherapy**

(71) Applicant: Pangaea Biotech, S.A., 08028 Barcelona (ES)
(72) Inventor: Tarón Roca, Miguel, 08916 Badalona (ES); Rosell Costa, Rafael, 08916 Badalona (ES)
(74) Representative: ABG Patentes, S.L.

(57) **Abstract**

The invention relates to methods for predicting the clinical outcome of a patient which suffers from bladder cancer based on the expression levels of BRCA1 wherein high BRCA1 expression levels are indicative of a poor prognosis. Moreover, the invention relates to methods for predicting the response to chemotherapy of a patient which suffers from bladder cancer based on the expression levels of BRCA1, in particular, in patients which have been treated with chemotherapy prior to surgical removal of the tumor.

SEQUENCE LISTING

<110>  PANGAEA BIOTECH, S.A.

<120>  BRCA1 mRNA EXPRESSION LEVELS PREDICT SURVIVAL IN PATIENTS WITH BLADDER
CANCER TREATED WITH CISPLATIN-BASED CHEMOTHERAPY

<130>  P3668EP00

<160>  6

<170>  PatentIn version 3.3

<210>  1
<211>  18
<212>  DNA
<213>  Artificial

<220>
<223>  PCR beta-actin forward primer

<400>  1
tgagcgcggc tacagctt                                                    18


<210>  2
<211>  22
<212>  DNA
<213>  Artificial

<220>
<223>  PCR beta-actin reverse primer

<400>  2
tccttaatgt cacgcacgat tt                                               22


<210>  3
<211>  18
<212>  DNA
<213>  Artificial

<220>
<223>  5' labeled fluorescent reporter dye (6FAM) probe for beta-actin

<400>  3
accaccacgg ccgagcgg                                                    18


<210>  4
<211>  26
<212>  DNA
<213>  Artificial

<220>
<223>  PCR BRCA1 forward primer

<400>  4
ggctatcctc tcagagtgac atttta                                           26


<210>  5
<211>  25
<212>  DNA
<213>  Artificial

<220>
<223>  PCR BRCA1 reverse primer

<400>  5
gctttatcag gttatgttgc atggt                                          25


<210>  6
<211>  15
<212>  DNA
<213>  Artificial

<220>
<223>  5' labeled fluorescent reporter dye (6FAM) probe for BRCA1

<400>  6
ccactcagca gaggg                                                     15



1

## Description

### FIELD OF THE INVENTION

The invention relates to the field of diagnostic and therapy, in particular to a method of providing personalized diagnosis and therapy to bladder cancer patients based on the expression of certain biomarkers in a sample from said patients.

### BACKGROUND OF THE INVENTION

Bladder cancer is one of the most common neoplasms, with more than 50,000 newly diagnosed cases in the United States alone each year. Most bladder cancers are transitional cell carcinomas (cancer that begins in cells that normally make up the inner lining of the bladder). Other types include squamous cell carcinoma (cancer that begins in thin, flat cells) and adenocarcinoma (cancer that begins in cells that make and release mucus and other fluids). The cells that form squamous cell carcinoma and adenocarcinoma develop in the inner lining of the bladder as a result of chronic irritation and inflammation.

Bladder cancer is usually treated by surgery (Transurethral resection, Radical cystectomy or segmental cystectomy), radiation therapy or chemotherapy. In patients with locally-advanced bladder cancer, neoadjuvant chemotherapy followed by cystectomy improves survival compared to surgery alone, especially in patients attaining a pathological response (pT0-1N0M0).

Bellmunt et al (Ann. Oncol., 2007, 522-528) describe methods for predicting survival of patients suffering advanced and metastatic bladder cancer in response to chemotherapy based on the expression levels of different genes involved in DNA repair. These authors could identify ERCC1 as prognostic marker but failed to observe any correlation between survival and expression levels of other genes such as RRM1, BRCA1 and caveolin-1.

Urushibara et al (Jpn J Clin Oncol., 2007, 37:56-61) have reported that expression levels of hsp60 can be used as independent factor for predicting survival of patients suffering from bladder cancer after cystectomy and neoadjuvant chemoradiotherapy.

Takata et al (Clin. Cancer Res., 2005, 11:2625-2636) have described 14 genes whose expression levels vary between invasive bladder tumors which respond to MVAC-based neoadjuvant chemotherapy and non-responding tumors.

Sarkis et al. (J.Clin.Oncol., 1995, 13:1384-1390) have described that patients carrying mutations in the p53 gene showed a poorer clinical outcome in response to MVAC-based neoadjuvant chemotherapy.

Inoue et al (Clin. Cancer Res., 2000, 6:4866-4873) have described that the expression levels of angiogenesis factor as detected by in situ hybridisation correlated with high recurrence and metastasis in patients with bladder cancer after surgical resection and MVAC neoadjuvant therapy.

However, there is still a need for further markers useful for predicting the clinical outcome of bladder cancer patients in response to cystectomy and neoadjuvant (preoperative) chemotherapy.

### SUMMARY OF THE INVENTION

In a first aspect, the invention relates to a method for predicting the clinical outcome of a patient suffering from bladder cancer comprising determining the expression levels of the BRCA1 gene in a sample from said patient wherein decreased expression levels of the BRCA1 gene in said sample when compared with reference levels are indicative of a good clinical outcome.

In a second aspect, the invention relates to a method for predicting whether a patient suffering from bladder cancer will respond to neoadjuvant chemotherapy which comprises determining the expression levels of BRCA1 gene in a sample from the patient wherein if the expression levels are lower than or similar to the reference values, then the patient is predicted to respond to the treatment with neoadjuvant chemotherapy.

### BRIEF DESCRIPTION OF THE FIGURES

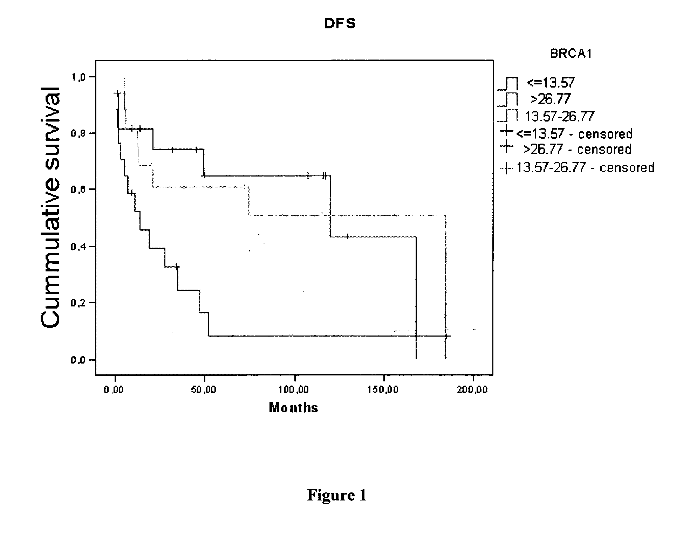

Figure 1. Kaplan-Meier curve showing accumulated survival of patients showing low (<=13.57), medium (13,57-26,77) and high (>26,77) expression levels of BRCA1 mRNA. The term "censored" indicates losses from the sample before the final outcome is observed.

### DETAILED DESCRIPTION OF THE INVENTION

The authors of the present invention have found that the clinical outcome of patients suffering from bladder cancer and wherein the bladder tumor has been surgically resected after preoperative (neoadjuvant) chemotherapy closely correlates with the expression levels of BRCA1. Thus, in a first aspect, the invention relates to a method for predicting the clinical outcome of a patient suffering from bladder cancer comprising determining the expression levels of the BRCA1 gene in a sample from said patient wherein decreased expression levels in said sample when compared with reference levels are indicative of a good clinical outcome.

The prediction of the clinical outcome can be done by using any endpoint measurements used in oncology and known to the skilled practitioner. Useful endpoint parameters to describe the evolution of a disease include:
- disease-free progression which, as used herein, describes the proportion of patients in complete remission who have had no recurrence of disease during the time period under study.
- objective response, which, as used in the present invention, describes the proportion of treated people in whom a complete or partial response is observed.
- tumor control, which, as used in the present invention, relates to the proportion of treated people in whom complete response, partial response, minor response or stable disease ≥ 6 months is observed.
- progression free survival which, as used herein, is defined as the time from start of treatment to the first measurement of cancer growth.
- six-month progression free survival or PFS6" rate which, as used herein, relates to the percentage of people wherein free of progression in the first six months after the initiation of the therapy and
- median survival which, as used herein, relates to the time at which half of the patients enrolled in the study are still alive.

The term "bladder cancer" relates to a tumour of the bladder and includes any histology subtype which typically appears in bladder cancer such as transitional cell carcinomas, squamous cell carcinoma and adenocarcinoma, any clinical subtype such as superficial, muscle-invasive or metastatic disease cancer and any TMN stage including T0-T4, N0-N4 and M0-M1 tumors.

The term "sample" as used herein, relates to any sample which can be obtained from the patient. The present method can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In a particular embodiment, said sample is a tumour tissue sample or portion thereof. In a more particular embodiment, said tumor tissue sample is a bladder tumor tissue sample from a patient suffering from bladder cancer. Said sample can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Tumour cells can additionally be obtained from fine needle aspiration cytology. In order to simplify conservation and handling of the samples, these can be formalin-fixed and paraffin-embedded or first frozen and then embedded in a cryosolidifiable medium, such as OCT-Compound, through immersion in a highly cryogenic medium that allows for rapid freeze.

The method of the invention requires determining the expression levels of the BRCA1 gene. In a preferred embodiment, the determination of the expression levels of the BRCA1 gene can be carried out by measuring the expression levels of the mRNA encoded by the BRCA1 gene. For this purpose, the biological sample may be treated to physically or mechanically disrupt tissue or cell structure, to release intracellular components into an aqueous or organic solution to prepare nucleic acids for further analysis. The nucleic acids are extracted from the sample by procedures known to the skilled person and commercially available. RNA is then extracted from frozen or fresh samples by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

In a particular embodiment, the expression level is determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene, for example. Deparaffinized samples can be rehydrated with an aqueous solution of a lower alcohol. Suitable lower alcohols, for example include, methanol, ethanol, propanols, and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample.

While all techniques of gene expression profiling (RT-PCR, SAGE, or TaqMan) are suitable for use in performing the foregoing aspects of the invention, the gene mRNA expression levels are often determined by reverse transcription polymerase chain reaction (RT-PCR). The detection can be carried out in individual samples or in tissue microarrays.

In order to normalize the values of mRNA expression among the different samples, it is possible to compare the expression levels of the mRNA of interest in the test samples with the expression of a control RNA. A "Control RNA" as used herein, relates to a RNA whose expression levels do not change or change only in limited amounts in tumor cells with respect to non-tumorigenic cells. Preferably, the control RNA are mRNA derived from housekeeping genes and which code for proteins which are constitutively expressed and carry out essential cellular functions. Preferred housekeeping genes for use in the present invention include β-2-microglobulin, ubiquitin, 18-S ribosomal protein, cyclophilin, GAPDH and actin. In a preferred embodiment, the control RNA is β-action mRNA. In one embodiment relative gene expression quantification is calculated according to the comparative Ct method using β-actin as an endogenous control and commercial RNA controls as calibrators. Final results, are determined according to the formula 2-(ΔCt sample-ΔCt calibrator), where ΔCT values of the calibrator and sample are determined by subtracting the CT value of the target gene from the value of the β-actin gene.

The determination of the level of expression of the BRCA1 gene needs to be correlated with the reference values which correspond to the median value of expression levels of BRCA1 measured in a collection of tumor tissue in biopsy samples from cancer patients, previous to the neoadjuvant chemotherapeutic treatment. Once this median value is established, the level of this marker expressed in tumor tissues from patients can be compared with this median value, and thus be assigned a level of "low," "normal" or "high". The collection of samples from which the reference level is derived will preferably be constituted from patient suffering from the same type of cancer. In any case it can contain a different number of samples. The use of a reference value used for determining whether the expression of a gene sample is "increased" or "decreased" corresponds to the median value of expression levels of BRCA1 measured in a RNA sample obtained by pooling equal amounts of RNA from each of the tumour samples obtained by biopsy from cancer patients previous to the neoadjuvant chemotherapeutic treatment. Once this median value is established, the level of this marker expressed in tumours tissues from patients can be compared with this median value, and thus be assigned a level of "increased" or "decreased." In a particular embodiment, an increase in expression above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "decreased" expression.

Due to inter-subject variability (e.g. aspects relating to age, race, etc.) it is very difficult (if not practically impossible) to establish absolute reference values for BRCA1. Thus, in a particular embodiment, the reference values for "increased" or "decreased" BRCA1 expression are determined by calculating percentiles by conventional means involving the testing of a group of samples isolated from normal subjects (i.e. people with no diagnosis of NSCLC) for the expression levels of the BRCA1 gene. The "increased" levels can then be assigned, preferably, to samples wherein expression levels for the BRCA1 genes is equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population.

In a preferred embodiment BRCA1 expression values are divided into terciles. As an example, real-time quantitative PCR was used to determine BRCA1 mRNA levels in 51 tumor biopsies from bladder cancer patients who had received cisplatin chemotherapy, and divided the gene expression values into terciles. When results were correlated with outcome (DFS and MS), it was observed that patients with BRCA1 levels in the lower tercile (tercile 1) had a significantly decreased risk of relapse (DFS) and a significantly better survival (MS) when compared to those in the top and middle terciles (see Figure 1 and table 1).

In another embodiment, the expression levels of the BRCA1 gene are determined by measuring the expression of the BRCA1 protein. The determination of the expression levels of the BRCA1 protein can be carried out by immunological techniques such as e.g. ELISA, Western Blot or immunofluorescence. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression and subcellular localization by microscopy. Generally, the cells under study are previously fixed with paraformaldehyde and permeabilised with a non-ionic detergent. ELISA is based on the use of antigens or antibodies labelled with enzymes so that the conjugates formed between the target antigen and the labelled antibody results in the formation of enzymatically-active complexes. Since one of the components (the antigen or the labelled antibody) are immobilised on a support, the antibody-antigen complexes are immobilised on the support and thus, it can be detected by the addition of a substrate which is converted by the enzyme to a product which is detectable by, e.g. spectrophotometry or fluorometry. This technique does not allow the exact localisation of the target protein or the determination of its molecular weight but allows a very specific and highly sensitive detection of the target protein in a variety of biological samples (serum, plasma, tissue homogenates, postnuclear supernatants, ascites and the like). In a preferred embodiment, the BRCA1 protein is detected by immunohistochemistry (IHC) analysis using thin sections of the biological sample immobilised on coated slides. The sections are then deparaffinised, if derived from a paraffinised tissue sample, and treated so as to retrieve the antigen. The detection can be carried out in individual samples or in tissue microarrays.

Any antibody or reagent known to bind with high affinity to the target protein can be used for detecting the amount of target protein. It is preferred nevertheless the use of antibody, for example polyclonal sera, hybridoma supernatants or monoclonal antibodies, antibody fragments, Fv, Fab, Fab' y F(ab')2, ScFv, diabodies, triabodies, tetrabodies and humanised antibodies.

In yet another embodiment, the determination of BRCA1 protein expression levels can be carried out by constructing a tissue microarray (TMA) containing the patient samples assembled, and determining the expression levels of BRCA1 protein by immunohistochemistry techniques. Immunostaining intensity can be evaluated by two different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumoral cells and the specific cut-off for each marker. As a general criterion, the cut-offs were selected in order to facilitate reproducibility, and when possible, to translate biological events.

The determination of the level of expression of the BRCA1 gene needs to be correlated with the reference values which correspond to the median value of expression levels of BRCA1 measured in a collection of tumor tissue in biopsy samples from cancer patients, previous to the neoadjuvant chemotherapeutic treatment. Once this median value is established, the level of this marker expressed in tumor tissues from patients can be compared with this median value, and thus be assigned a level of "low," "normal" or "high."

The authors of the present invention have also found that the degree of lymph node involvement, i.e. lymphatic invasion, can be used together with the values of BRCA1 expression levels, as a predictive marker for the clinical outcome of patients suffering from bladder cancer who have been treated with a cisplatin-based neoadjuvant therapy. Therefore, in another embodiment the method of the invention further comprises measuring lymph node involvement, wherein if lymph node involvement is negative, it is indicative of good clinical outcome.

The expression "lymph node involvement" as used herein, is understood as the spread of the tumor cells to the lymph nodes and blood vessels located in the vicinity of the tissue which contains the tumor and is determined using standard procedures known to the skilled person.

In another embodiment, the method of the invention is applied to a patient which has undergone a surgical resection of the tumor. In a still more preferred embodiment, the patient which suffers bladder cancer and whose clinical outcome is to be predicted is treated with preoperative (neoadjuvant) therapy. Suitable neoadjuvant therapies for bladder cancer include radiotherapy, chemotherapy or chemoradiotherapy. In a preferred embodiment, the neoadjuvant therapy is chemotherapy. Preferrably, the neoadjuvant chemotherapy is a cisplatin-based chemotherapy and, more preferably, the cisplatin-based neoadjuvant chemotherapy is selected from the group of cisplatin, cisplatin-ethotrexate-vinblastine, gemcitabine-cisplatin, methotrexate-vinblastine-doxorubicin-cisplatin (MVAC), cyclophosphamide-doxorubicin-cisplatin (CISCA), dose-dense MVAC (ddMVAC), cisplatin-adriamycin (CA), cisplatin-methotrexate (CM), cisplatin-fluorouracil (CF) and methotrexate-vinblastine-epirubicin-cisplatin (MVEC).

The authors of the present invention have also found that the degree of lymphatic invasion can be used as a predictive marker of the clinical outcome of patients suffering from bladder cancer. Thus, in another aspect, the invention relates to a method for predicting the clinical outcome of a patient suffering from bladder cancer comprising determining the lymphatic invasion in said patient wherein increased lymphatic invasion is indicative of a poor clinical outcome.

The patient whose clinical outcome is to be determined on the basis of the degree of lymphatic invasion can be a patient which has undergone surgical resection of the tumor and, preferably, a patient which has been treated with neoadjuvant therapy. Suitable neoadjuvant therapies are those mentioned above in relation to the method for predicting the clinical outcome based on determination of the expression levels of the BRCA1 gene. The clinical outcome can be measured using any of the parameters mentioned above (disease-free progression, objective response, tumor control, progression free survival, median survival, six-month progression free survival, overall survival and the like).

The findings of the inventors allow the development of personalised therapies for patients suffering from bladder cancer wherein the expression or BRCA1 correlates with the possibility that the patient will respond to neoadjuvant chemotherapy. Thus, in another aspect, the invention relates to a method for predicting whether a patient suffering from bladder cancer will respond to neoadjuvant chemotherapy which comprises determining the expression levels of BRCA1 gene in a sample from the patient wherein if the expression levels are lower than or similar to the reference values, then the patient is predicted to respond to the treatment with neoadjuvant chemotherapy.

The term "sample" has been previously defined and can be applied to any type of biological sample from a patient, such as a biopsy sample, tissue, cell or fluid (serum, saliva, semen, sputum, cerebral spinal fluid (CSF), tears, mucus, sweat, milk, brain extracts and the like). In a particular embodiment, said sample is a tumour tissue sample or portion thereof. In a more particular embodiment, said tumor tissue sample is a bladder tumor tissue sample from a patient suffering from bladder cancer or a formalin embedded bladder tissue sample.

The determination of the expression levels of the BRCA1 gene is carried out by measuring the expression levels of the mRNA encoded by the BRCA1 gene or by measuring the expression levels of the BRCA1 gene product using any of the procedures previously mentioned. The values must be normalised using a control housekeeping mRNA or protein and compared to reference values which correspond to the median value of expression levels of BRCA1 measured in a collection of tumor tissue in biopsy samples from cancer patients, previous to the neoadjuvant chemotherapeutic treatment. Once this median value is established, the level of this marker expressed in tumor tissues from patients can be compared with this median value, and thus be assigned a level of "low," "normal" or "high".

The authors of the present invention have also found that the degree of lymph node involvement, i.e. lymphatic invasion can be used, in combination with the BRCA1 gene expression levels, to increase the reliability of the method for predicting whether a patient suffering from bladder cancer will respond to neoadjuvant chemotherapy. Thus, in another embodiment, the method for predicting whether a patient suffering from bladder cancer will respond to neoadjuvant chemotherapy further comprises measuring lymph node involvement, wherein if lymph node involvement is negative, then the patient is predicted to respond to the treatment with neoadjuvant chemotherapy.

In another embodiment, the neoadjuvant chemotherapeutic which can be used once a patient has been selected as candidate for treatment with neoadjuvant chemotherapy is perferrably a cisplatin-based chemotherapy. More preferably, the cisplatin-based neoadjuvant chemotherapy is selected from the group of cisplatin, cisplatin-methotrexate-vinblastine, gemcitabine-cisplatin, methotrexate-vinblastine-doxorubicin-cisplatin (MVAC), cyclophosphamide-doxorubicin-cisplatin (CISCA), dose-dense MVAC (ddMVAC), cisplatin-adriamycin (CA), cisplatin-methotrexate (CM), cisplatin-fluorouracil (CF) and methotrexate-vinblastine-epirubicin-cisplatin (MVEC).

The following examples are provided as merely illustrative and are not to be construed as limiting the scope of the invention.

### EXAMPLES

### Methods

BRCA1 mRNA expression levels were analysed by quantitative PCR in tumor biopsies obtained by transurethral resection from 51 patients with locally-advanced bladder cancer. 44 patients were clinically staged as cT3-4N0M0, 5 patients had regional lymph nodes (cTxN+), and 2 patients had distant lymph nodes. 35 patients received cisplatin, methotrexate plus vinblastine; 16 patients were treated with gemcitabine plus cisplatin. The BRCA1 gene expression was measured as previously described by Specht K, et al. (2001) (Am. J. Pathol., 158, 419-429 and Krafft AE, et al. (1997) Mol. Diagn. 3, 217-230. After standard tissue sample deparaffinization using xylene and alcohols, samples were lysed in a Tris-chloride, EDTA, sodium dodecyl sulphate (SDS) and proteinase K containing buffer. RNA was then extracted with phenol-chloroform-isoamyl alcohol followed by precipitation with isopropanol in the presence of glycogen and sodium acetate. RNA was resuspended in RNA storage solution (Ambion Inc; Austin TX, USA) and treated with DNAse I to avoid DNA contamination. cDNA was synthesized using M-MLV retrotranscriptase enzyme. Template cDNA was added to Taqman Universal Master Mix (AB; Applied Biosystems, Foster City, CA, USA) in a 12.5-µl reaction with specific primers and probe for each gene. The primer and probe sets were designed using Primer Express 2.0 Software (AB). Quantification of gene expression was performed using the ABI Prism 7900HT Sequence Detection System (AB). Primers and probe for BRCA1 mRNA expression analysis were designed according to the Ref Seq NM_007294 (http://www.ncbi.nlm.nih.gov/LocusLink). Forward primer is located in exon 8 (position 4292 bp to 4317 bp), reverse primer in exon 9 (position 4336 bp to 4360 bp), and probe in the exon 8/9 junction (position 4313 bp to 4333 bp). The PCR product size generated with these primers was 69 bp. The primers and 5'labeled fluorescent reporter dye (6FAM) probe were as follows: β-actin: forward 5' TGA GCG CGG CTA CAG CTT 3', reverse 5' TCC TTA ATG TCA CGC ACG ATT T 3', probe 5' ACC ACC ACG GCC GAG CGG 3'; BRCA1: forward 5'GGC TAT CCT CTC AGA GTG ACA TTT TA 3', reverse 5' GCT TTA TCA GGT TAT GTT GCA TGG T 3', probe 5' CCA CTC AGC AGA GGG 3'. Relative gene expression quantification was calculated according to the comparative Ct method using β-actin as an endogenous control and commercial RNA controls (Stratagene, La Jolla, CA) as calibrators. Final results, were determined as follows: 2^{-(ΔCt sample-ΔCt calibrator)}, where ΔC_{T} values of the calibrator and sample are determined by subtracting the C_{T} value of the target gene from the value of the β-actin gene. In all experiments, only triplicates with a standard deviation (SD) of the Ct value <0.20 were accepted. In addition, for each sample analyzed, a retrotranscriptase minus control was run in the same plate to assure lack of genomic DNA contamination.

### Results

After a median of 3 cycles of chemotherapy, cystectomy was performed in 49 patients (96%). A significant pathological response (pT0 or T1) was attained in 25 patients (50%). A close correlation was found between BRCA1 levels and pathological response. 21 patients (65%) with low/intermediate BRCA1 levels but only 4 p (26%) with high BRCA levels obtained a pathological response (P=0.01). Median disease-free survival (DFS) was 120 months in 17 patients with low BRCA1 levels, 184 months in 17 patients with intermediate BRCA1 levels, and only 14 months in 17 patients with high BRCA1 levels (P=0.02). In the multivariate analysis of DFS, only BRCA1 levels and lymphatic invasion emerged as independent prognostic markers (see Table 2).

**Table 1:**

| BRCA1 | Average | | | | Mean | | | |
|---|---|---|---|---|---|---|---|---|
| | Estimate | Typical error | 95% CI | | Estimate | Typical error | 95% CI | |
| | | | Lower limit | Upper limit | | | Lower limit | Upper limit |
| <=13.57 | 104,974 | 20,801 | 64,024 | 145,744 | 120,000 | 64,691 | ,000 | 246,795 |
| 13,57-26,77 | 105,318 | 23,566 | 59,129 | 151,508 | 184,000 | ,000 | . | . |
| >26,77 | 31,827 | 13,111 | 6,129 | 57,524 | 14,000 | 7,561 | ,000 | 28,820 |
| Global | 80,466 | 12,243 | 56,470 | 104,461 | 47,000 | 15,636 | 16,354 | 77,646 |

| | | | | | | | | |
|---|---|---|---|---|---|---|---|---|
| Overall survival in univariant analysis of BRCA1 expression levels and disease-free survival | | | | | | | | |

**Table 2:**

| | HR | 95% CI | P |
|---|---|---|---|
| BRCA1 | | | |
| ≤13.57 | 1(ref.) | | |
| 13.57-26.77 | 1.16 | 0.4-3.36 | 0.77 |
| >26.77 | 3.1 | 1.15-8.40 | 0.02 |
| Lymphatic | | | |
| invasion | 7.8 | 2.54-23.90 | <0.0001 |
| Yes | 1(ref.) | | |
| No | | | |
| Diff. grade | | | |
| Low | 1(ref.) | | |
| High | 1.29 | 0.15-10.83 | 0.81 |

| | | | |
|---|---|---|---|
| Multivariant analysis of DFS including the BRCA1 mRNA levels, lymphatic invasion and differentiation grade. | | | |

**Table 3**

| | HR | 95% CI | P |
|---|---|---|---|
| BRCA1 | | | |
| ≤13.57 | 1(ref.) | | |
| 13.57-26.77 | 1.1 | 0.32-3.63 | 0.90 |
| >26.77 | 2.63 | 0.89-7.65 | 0.07 |
| Lymphatic invasion | | | |
| Yes | 9.8 | 3.03-31.52 | <0.0001 |
| No | 1(ref.) | | |
| Diff. Grade | | | |
| Low | 1(ref.) | | |
| High | 1.83 | 0.21-16.26 | 0.59 |

| | | | |
|---|---|---|---|
| Multivariant analysis of survival including BRCA1 mRNA levels, lymphatic invasion and differentiation grade. | | | |

## Claims

1. Method for predicting the clinical outcome of a patient suffering from bladder cancer comprising determining the expression levels of the BRCA1 gene in a sample from said patient wherein decreased expression levels of the BRCA1 gene in said sample when compared with reference levels are indicative of a good clinical outcome.

2. Method according to claims 1 or 2 wherein the sample is a tumor biopsy.

3. Method according to claims 1 or 2 further comprising measuring lymph node involvement wherein if lymph node involvement is negative, it is indicative of good clinical outcome.

4. Method according to any of claims 1 to 3 wherein the expression levels of the BRCA1 gene are determined by measuring the levels of mRNA encoded by the BRCA1 gene or the level of BRCA1 protein.

5. Method according to any of claims 1 to 4 wherein the patient has undergone surgical resection of the tumor.

6. Method according to claim 5 wherein the patient has been treated with neoadjuvant therapy.

7. Method according to claim 6 wherein the neoadjuvant therapy is chemotherapy.

8. Method according to claim 7 wherein said neoadjuvant chemotherapy is a cisplatin-based chemotherapy.

9. Method according to claim 8 wherein the cisplatin-based chemotherapy is selected from the group of cisplatin, cisplatin-methotrexate-vinblastine, gemcitabine-cisplatin, methotrexate-vinblastine-doxorubicin-cisplatin (MVAC), cyclophosphamide-doxorubicin-cisplatin (CISCA), dose-dense MVAC (ddMVAC), cisplatin-adriamycin (CA), cisplatin-methotrexate (CM), cisplatin-fluorouracil (CF) and methotrexate-vinblastine-epirubicin-cisplatin (MVEC).

10. Method according to any of claims 1 to 9 wherein the prediction of the clinical outcome is measured as disease-free survival and overall survival.

11. Method for predicting whether a patient suffering from bladder cancer will respond to neoadjuvant therapy which comprises determining the expression levels of BRCA1 gene in a sample from the patient wherein if the expression levels are lower than or similar to the reference values, then the patient is predicted to respond to the treatment with neoadjuvant chemotherapy.

12. Method according to claim 11 wherein the sample is a tumor biopsy.

13. Method according to claims 11 or 12 further comprising measuring lymph node involvement wherein if lymph node involvement is negative, then the patient is predicted to respond to the treatment with neoadjuvant chemotherapy.

14. Method according to any of claims 11 to 13 wherein the expression levels of the BRCA1 gene are determined by measuring the levels of mRNA encoded by the BRCA1 gene or the levels of BRCA1 protein.

15. Method according to claim 14 wherein the neoadjuvant therapy is chemotherapy.

16. Method according to claim 15 wherein said neoadjuvant chemotherapy is a cisplatin-based chemotherapy.

17. Method according to claim 16 wherein the cisplatin-based chemotherapy is selected from the group of cisplatin, cisplatin-methotrexate-vinblastine, gemcitabine-cisplatin, methotrexate-vinblastine-doxorubicin-cisplatin (MVAC), cyclophosphamide-doxorubicin-cisplatin (CISCA), dose-dense MVAC (ddMVAC), cisplatin-adriamycin (CA), cisplatin-methotrexate (CM), cisplatin-fluorouracil (CF) and methotrexate-vinblastine-epirubicin-cisplatin (MVEC).
